# EUROPÄISCHE PATENTSCHRIFT

(11) **EP 3 322 472 B1**
(45) Veröffentlichungstag und Bekanntmachung des Hinweises auf die Patenterteilung: **13.03.2019**
(21) Anmeldenummer: 16751466.0
(22) Anmeldetag: 16.06.2016
(51) Int. Cl.: A61M 35/00, A61N 1/32

(54) **BEHANDLUNGSGERÄT ZUR BEHANDLUNG MIT EINEM DIELEKTRISCH BEHINDERTEN PLASMA**
TREATMENT DEVICE FOR TREATMENT USING A DIELECTRIC-BARRIER PLASMA
APPAREIL DE TRAITEMENT POUR LE TRAITEMENT PAR UN PLASMA À BARRIÈRE DIÉLECTRIQUE

(30) Priorität: 14.07.2015 DE 102015111401
(43) Veröffentlichungstag der Anmeldung: 23.05.2018
(73) Patentinhaber: Cinogy GmbH, 37115 Duderstadt (DE)
(72) Erfinder: HAHNL, Mirko, 37339 Berlingerode (DE); STORCK, Karl-Otto, 37115 Duderstadt (DE); TRUTWIG, Leonhard, 37115 Duderstadt / Gerlingerode (DE); WANDKE, Dirk, 37308 Heilbad Heiligenstadt (DE); KOPP, Matthias, 37434 Gieboldehausen (DE); HELMKE, Andreas, 37574 Einbeck (DE)
(74) Vertreter: Lins, Martina
(86) Internationale Anmeldenummer: PCT/DE2016/100274
(87) Internationale Veröffentlichungsnummer: WO 2017/008781

(56) Entgegenhaltungen:
- EP-A1- 2 170 022
- DE-A1-102012 015 482
- DE-A1-102013 019 058
- US-A1- 2013 345 620
- US-A1- 2015 151 135

## Beschreibung

Die Erfindung betrifft ein Behandlungsgerät für eine mit einem dielektrisch behinderten Plasma zu behandelnde Oberfläche, mit einem Gehäuse, das eine Stirnwand aufweist, mit einer zur zu behandelnden Oberfläche durch ein wenigstens einen Teil der Stirnwand bildendes Dielektrikum abgeschirmten Elektrode, die mit einem Hochspannungsgenerator verbindbar ist, wobei die Stirnwand wenigstens einen Abstandshalter aufweist, mit dem beim Anliegen des wenigstens eines Abstandshalters an der zu behandelnden Oberfläche wenigstens ein Gasraum gebildet wird, in dem sich für die Behandlung das dielektrisch behinderte Plasma ausbildet.

Durch DE 10 2009 060 627 B4 ist eine Elektrodenanordnung aus einer flächigen flexiblen Elektrode und einem flexiblen flächigen Dielektrikum bekannt, bei dem das Dielektrikum die flächige Elektrode allseitig umgibt und lediglich ein Anschluss der Elektrode isolierend aus dem Dielektrikum zur Verbindung mit einem Hochspannungsgenerator herausgeführt ist. Das Dielektrikum ist zur Anlage an der zu behandelnden Oberfläche, beispielsweise Hautoberfläche eines menschlichen oder tierischen Körpers, vorgesehen und weist auf der Anlageseite eine Noppenstruktur auf, die als Abstandshalter fungiert, weil sich zwischen den Noppen Gasräume bilden, in denen das dielektrisch behinderte Plasma ausgebildet werden kann.

Mit einer ähnlichen Elektrodenanordnung ist ein Behandlungsgerät gemäß DE 10 2012 015 482 A1 ausgestattet, wobei das die Elektrode einbettende Dielektrikum die Stirnwand eines Gehäuses eines Behandlungsgeräts bildet. Die flexible Elektrodenanordnung aus dem flexiblen Dielektrikum mit der flexibel eingebetteten flächigen Elektrode wird dabei durch ein hinter der Elektrodenanordnung angeordnetes elastisches Andruckmittel gegen die zu behandelnde Oberfläche gedrückt, wodurch die Anpassbarkeit der Elektrodenanordnung an Konturen der zu behandelnden Oberfläche, insbesondere Hautoberfläche, verbessert wird.

Insbesondere für kosmetische und medizinische Zwecke wird eine Behandlung mit einem dielektrisch behinderten Plasma unterstützt durch ein aufgetragenes Behandlungsmittel. Bei einer kosmetischen Behandlung einer Hautoberfläche bewirkt die Behandlung mit dem dielektrisch behinderten Plasma sowohl eine Reinigung/Desinfizierung der Oberfläche als auch eine verbesserte Durchblutung und Aufweitung der Poren, sodass ein Behandlungsmittel effizient von der Haut aufgenommen werden kann. Im nicht kosmetischen Bereich kann beispielsweise auf eine Holz- oder Kunststoffoberfläche nach einer Plasmabehandlung verbessert ein Behandlungsmittel, wie beispielsweise, eine Grundierung oder Imprägnierung auf-gebracht werden, da sich die Haftung dieses Behandlungsmittels auf der Oberfläche verbessert. Es ist üblich, das Behandlungsmittel vor oder nach der Plasmabehandlung in einem getrennten Schritt aufzutragen.

Durch US 2015/0151135 A1 ist es bekannt, insbesondere mit einem Handgerät die Applikation eines dielektrisch behinderten Plasmas mit der Zugabe eines Vakzins zu kombinieren. Das Handgerät ist hierfür mit einem spritzenähnlichen zylindrischen Körper versehen, aus dem das Vakzin mit einem mit einer nach oben aus dem Gerät herausragenden Kolbenstange verbundenen Kolben herausdrückbar ist. Das Vakzin gelangt durch eine zentrale Öffnung in eine unterhalb einer ringförmigen Elektrode angeordneten Behandlungskammer und soll dort auf den zu behandelnden Körperteil einwirken. Die Einwirkung wird durch eine vorhergehende und/oder anschließende Plasmabehandlung unterstützt. Die Plasmabehandlung(en) und die Zugabe des Vakzins laufen somit nacheinander ab, wobei sich die Dauer der Plasmabehandlungen über wenige Minuten und die Einwirkzeit des Vakzins über mehrere Stunden erstrecken kann.

Der vorliegenden Erfindung liegt die Aufgabe zugrunde, ein Behandlungsgerät der eingangs erwähnten Art so auszubilden, dass eine verbesserte Behandlung durch ein dielektrisch behindertes Plasma unter Verwendung eines Behandlungsmittels, insbesondere eine kosmetische Behandlung der Hautoberfläche, möglich ist.

Zur Lösung dieser Aufgabe ist erfindungsgemäß ein Behandlungsgerät der eingangs erwähnten Art dadurch gekennzeichnet, dass auf der zu behandelnden Oberfläche abgewandten Seite der Stirnwand eine mit einem Behandlungsmittel füllbare ringförmige Vorratskammer angeordnet ist, dass die Stirnwand eine Vielzahl von Durchgangsöffnungen aufweist und dass die Vorratskammer in ihrem Volumen so verkleinerbar ist, dass bei der Verkleinerung des Volumens Behandlungsmittel durch die Durchgangsöffnungen in den Bereich der zu behandelnden Oberfläche gelangt.

Das erfindungsgemäße Behandlungsgerät ermöglicht somit das Auftragen des Behandlungsmittels auf die zu behandelnde Oberfläche auch während der Behandlung mit dem dielektrisch behinderten Plasma, sodass die Einwirkung von Plasma und Behandlungsmittel auf die zu behandelnde Oberfläche gleichzeitig und mit einem kontinuierlichen Nachschub des Behandlungsmittels erfolgen kann.

In einer bevorzugten Ausführungsform der Erfindung wird das Ausbringen des Behandlungsmittels, also die Verkleinerung der Vorratskammer, durch Ausübung eines Drucks auf die zu behandelnde Oberfläche mittels des Gehäuses vorgenommen. Das Aufbringen des Behandlungsmittels kann daher gleichmäßig bei einer gleichmäßigen Druckausübung auf die zu behandelnde Fläche erfolgen.

In einer ersten, hierfür geeigneten konstruktiven Ausbildung weist das Gehäuse teleskopisch ineinander greifende Mantelwandabschnitte auf, die durch Druck auf das Gehäuse in Richtung der zu behandelnden Oberfläche ineinander schiebbar sind, wodurch das Behandlungsmittel durch die Durchgangsöffnungen der Stirnwand austritt.

In einer weiteren konstruktiven Ausgestaltung dieses Prinzips ist die Stirnwand wenigstens teilweise flexibel ausgebildet und verformt sich durch einen Druck auf das Gehäuse in Richtung der zu behandelnden Oberfläche in Richtung des Inneren der Vorratskammer, wodurch die Volumenverkleinerung zur Ausbringung des Behandlungsmittels erfolgt.

In einer weiteren konstruktiven Ausführungsform weist das Gehäuse die Vorratskammer begrenzende flexible Mantelwände auf, die zur Verkleinerung des Volumens der Vorratskammer eindrückbar sind. Das Eindrücken der Mantelwände kann dabei sowohl durch einen radial nach innen gerichteten Druck auf die Mantelwände als auch durch einen axialen Druck in Richtung auf die zu behandelnde Oberfläche erfolgen, wenn dadurch die Mantelwand nach innen einbeult.

Durch die Volumenverkleinerung der Vorratskammer wird das Behandlungsmittel durch die Durchgangsöffnungen der Stirnwand des Gehäuses in den Bereich der zu behandelnden Oberfläche gedrückt. Ersichtlich muss hierfür das Behandlungsmittel fließfähig sein. Dies ist der Fall, wenn das Behandlungsmittel in geeigneter Form pulverförmig, pastös, gasförmig oder flüssig ist. Zweckmäßigerweise sind die lichten Weiten der Durchgangsöffnungen dem entsprechend angepasst. Bei dem Ausdrücken eines pulverförmigen Behandlungsmittels sind größere Durchgangsöffnungen verwendbar, als für pastöse oder zähflüssige Behandlungsmittel, während dünnflüssigere oder gasförmige Behandlungsmittel sinnvollerweise über Durchgangsöffnungen mit einem geringen lichten Querschnitt ausgebracht werden.

In einer konstruktiven Ausbildung des erfindungsgemäßen Behandlungsgeräts ist die Elektrode als flächige Elektrode in das flächig ausgebildete Dielektrikum allseitig eingebettet. Da das Dielektrikum die flächige Elektrode allseitig umgibt, muss auch die Elektrode Durchgangsöffnungen aufweisen. Diese sind aber erfindungsgemäß größer als die Durchgangsöffnungen des Dielektrikums, sodass im Dielektrikum Durchgangskanäle gebildet werden, die durchgehend radial von dem Dielektrikum begrenzt sind, sodass ein unerwünschter Stromfluss über das Behandlungsmittel sicher vermieden wird.

Für die Erfindung kann es zweckmäßig sein, wenn die Anordnung aus Dielektrikum und eingebetteter Elektrode flexibel ausgebildet ist.

Das Dielektrikum kann sich über die gesamte Stirnwand erstrecken, sodass die Stirnwand durch das Dielektrikum gebildet ist. In diesem Fall ist die flexible Ausbildung der Anordnung aus Dielektrikum und eingebetteter Elektrode vorteilhaft, um eine Anpassung an nicht ebene Konturen der zu behandelnden Oberfläche zu ermöglichen.

In einer anderen konstruktiven Ausgestaltung der Erfindung ist die Stirnwand wenigstens zweiteilig ausgebildet. Insbesondere kann dabei ein erstes Teil der Stirnwand durch das die Elektrode abschirmende Dielektrikum gebildet sein und wenigstens ein zweiter Teil der Stirnwand die Vorratskammer begrenzen und die Durchgangsöffnungen aufweisen. Auch für diese Ausführungsform sind die oben beschriebenen Möglichkeiten der Verkleinerung des Volumens der Vorratskammer zum Ausbringen des Behandlungsmittels alle einsetzbar und können nach Anwendungsfall und Zweckmäßigkeit ausgewählt werden.

Es kann vorteilhaft sein, wenn das Dielektrikum einen zentralen Abschnitt der Stirnwand bildet und der zweite Teil der Stirnwand das Dielektrikum - und damit die in das Dielektrikum eingebettete Elektrode - ringförmig umgibt. In diesem Fall kann es ausreichend sein, wenn der zweite Teil der Stirnwand flexibel ist, während die zentrale Anordnung aus dem Dielektrikum und der von dem Dielektrikum abgeschirmten Elektrode hart, also bei den in der Praxis für die Behandlung aufgewandten Drücken unflexibel ist.

Der wenigstens eine zweite Teil der Stirnwand kann aus einem isolierenden Kunststoff bestehen, also quasi das Dielektrikum in der Stirnwand auch außerhalb des Bereichs der Elektrode forstsetzen. Bei dieser konstruktiven Ausgestaltung ist die Ausbildung des wenigstens einen zweiten Teils der Stirnwand aus einem isolierenden Material nicht zwingend erforderlich, sodass auch Materialien mit einer mittleren oder hohen Leitfähigkeit einsetzbar sind.

In dem Fall kann es zweckmäßig sein, wenn der wenigstens eine zweite Teil der Stirnwand den wenigstens einen Abstandshalter ausbildet, mit dem die Anordnung aus Dielektrikum und Elektrode in einem geringen Abstand zur zu behandelnden Oberfläche gehalten wird, wenn der wenigstens eine Abstandshalter auf der zu behandelnden Oberfläche aufliegt. Dadurch bildet sich ein kleiner Zwischenraum zwischen dem die Elektrode abschirmenden Dielektrikum und der zu behandelnden Oberfläche aus. In diesem Gasraum bzw. Luftraum entsteht das dielektrisch behinderte Plasma durch Ionisierung des dort vorhandenen Gases bzw. der Luft, wobei ein direkter Stromfluss zwischen der zu behandelnden Oberfläche und der Elektrode durch das Dielektrikum verhindert wird, sodass für die Plasmaausbildung nur Verschiebeströme möglich sind. Dies gilt natürlich auch für die Anordnung, bei der das Dielektrikum selbst mit wenigstens einem Abstandshalter ausgebildet ist, sodass sich ein Gasraum bzw. Luftraum dieser Art zwischen dem vertieften Bereich des Dielektrikums (außerhalb des wenigstens einen Abstandshalters) und der zu behandelnden Oberfläche besteht. Auch hierbei wird ein direkter Stromfluss zwischen der Elektrode und der zu behandelnden Oberfläche verhindert.

Die Plasmaausbildung kann mit einer Gleichhochspannung erfolgen, wobei nur ein anfänglicher Verschiebestrom entsteht und die Potentialdifferenz das Plasma aufrechterhält. Bevorzugt ist jedoch die Anwendung einer Wechselhochspannung, wobei das Hochspannungspotential zwischen einer positiven und einer negativen Spannung wechselt. Bevorzugt ist dabei, dass die zu behandelnde Oberfläche, beispielsweise die Hautoberfläche bzw. der Körper des Lebewesens, als so genannte floatende Gegenelektrode fungiert, die dem Potentialwechsel der Wechselspannung nur träge folgen könnte und daher aufgrund der Wechselfrequenz auf einem Mittenpotential, das das Massepotential sein wird, im Wesentlichen verharrt.

Es ist zweckmäßig, das Behandlungsmittel nicht unmittelbar in die Vorratskammer einzufüllen, insbesondere wenn ein pastöses oder flüssiges Behandlungsmittel verwendet wird. In diesem Fall ist es zweckmäßig, das Behandlungsmittel in einem Trägermaterial in die Vorratskammer einzubringen. Das Trägermaterial kann ein beliebiges lockeres, durch die Volumenverkleinerung der Vorratskammer zusammendrückbares Material sein, wie beispielsweise ein Wattematerial, ein Vliesmaterial oder ein offenporiges Schwammmaterial. Das Behandlungsmittel wird dabei aus dem Trägermaterial herausgepresst, wenn das Volumen der Vorratskammer in wenigstens einer der beschriebenen Arten verkleinert wird.

Eine besonders vorteilhafte Ausführungsform der Erfindung ergibt sich daraus, dass ein Gehäuseteil aus Stirnwand und zumindest einem Teil der Vorratskammer als auswechselbares Kopfstück ausgebildet ist. Das Kopfstück nimmt dabei das Behandlungsmittel in einer Menge auf, die für eine Behandlung der Oberfläche vorgesehen ist, beispielsweise für eine kosmetische Gesichtsbehandlung. Nachdem das Behandlungsmittel aufgebraucht und die Behandlung beendet ist, kann das auswechselbare Kopfstück entfernt werden und das erfindungsgemäße Behandlungsgerät durch ein neues Kopfstück wieder einsatzfähig gemacht werden. Auf diese Weise ist ein hoher Hygienestandard gewährleistet, der keine oder nur geringe und einfach durchzuführende Reinigungsmaßnahmen erfordert.

Die Erfindung soll im Folgenden anhand von in den Zeichnungen dargestellten Ausführungsbeispielen näher erläutert werden. Es zeigen:
- Figur 1: eine Ansicht einer ersten Ausführungsform eines erfindungsgemäßen Behandlungsgeräts im montierten Zustand;

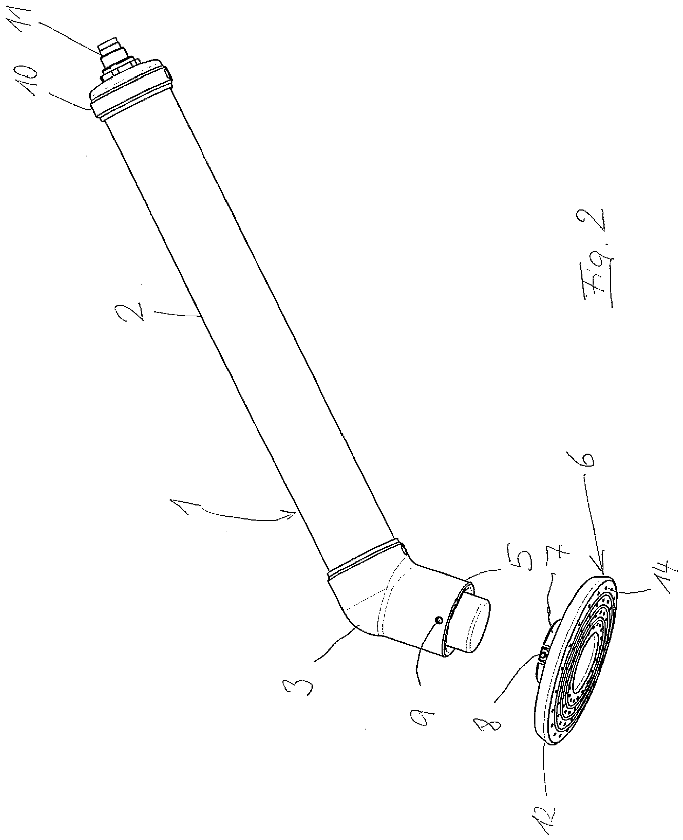

- Figur 2: die Ansicht gemäß Figur 1 mit abgenommenen Kopfstück;
- Figur 3: eine Schnittdarstellung des Behandlungsgeräts gemäß Figur 2;
- Figur 4: eine Schnittdarstellung des vollständig montierten Behandlungsgeräts gemäß Figur 1;
- Figur 5: eine vergrößerte Darstellung des Aufbaus des Kopfstücks und eines Endes des übrigen Gehäuses in separater Position;
- Figur 6: eine Darstellung gemäß Figur 5 nach dem Einsetzen des Kopfstücks in das übrige Gehäuse;
- Figur 7: eine Schnittdarstellung eines Kopfstücks eines Behandlungsgeräts nach einer zweiten Ausführungsform der Erfindung;
- Figur 8: eine vergrößerte Ausschnittsdarstellung des unteren Teils des Kopfstücks gemäß Figur 7;
- Figur 9: eine explodierte Darstellung des Kopfstücks gemäß Figur 7;
- Figur 10: eine perspektivische Draufsicht auf ein Zwischenstück mit einem Mantelwandungsabschnitt des Gehäuses;
- Figur 11: eine Draufsicht auf die Stirnwand des Behandlungsgeräts gemäß Figur 7;
- Figur 12: eine perspektivische Ansicht des Kopfstücks gemäß Figur 7.

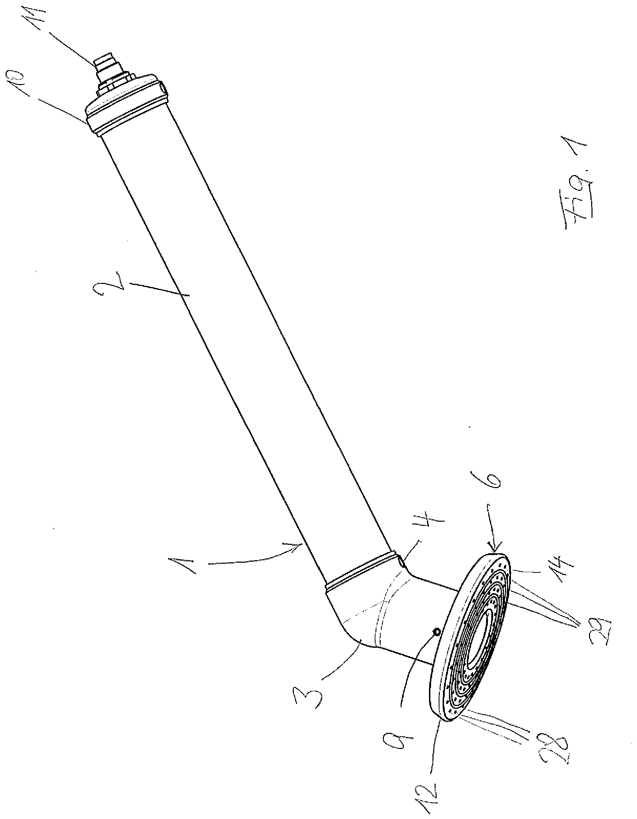

Das in Figur 1 dargestellte Behandlungsgerät gemäß einer ersten Ausführungsform der Erfindung weist ein Gehäuse 1 auf, das aus einem Griffstück 2 und einem Winkelstück 3 besteht. Beide Gehäuseteile, sind als Hohlrohre ausgebildet, wobei das Griffstück 2 ein geradliniges Hohlrohr ist und das Winkelstück 3 zwei über einen Winkel von etwa 135° miteinander verbundene Rohrenden aufweist. Griffstück 2 und Winkelstück 3 sind dadurch miteinander verbunden, dass das Winkelstück 3 eine Einschubaufnahme für das Hohlrohr des Griffstücks 2 aufweist. Das in das Winkelstück 3 eingeschobene Griffstück 2 ist mit einer Schraube 4 im montierten Zustand fixiert. Das Griffstück 2 kann als gezogenes Rohr aus Kunststoff oder Metall gebildet sein, während das Winkelstück 3 vorzugsweise ein Spritzgussteil ist. In das vom Griffstück 2 wegzeigende Ende 5 des Winkelstücks 3 ist ein Kopfstück 6 mit einem rohrförmigen Ansatz 7 einschiebbar. Der rohrförmige Ansatz 7 weist zwei diametral gegenüberliegende federnde Raststifte 8 auf, die in jeweils eine Rastöffnung 9 des rohrförmigen Endes 5 des Griffstücks 2 zur Fixierung des Kopfstücks 6 am Winkelstück 3 einrasten können, wodurch eine gesicherte Verbindung in axialer Richtung und bezüglich einer Rotation hergestellt wird.

Das nicht mit dem Winkelstück 3 verbundene Ende des Griffstücks 2 ist durch eine Stirnkappe 10 mit einer Kabeldurchführung 11 abgeschlossen. Vorzugsweise ist die Stirnkappe 10 auf ein Gewinde des Griffstücks 2 aufgeschraubt.

Das Kopfstück 6 weist eine Mantelwand 12 auf, die an den rohrförmigen Ansatz 7 über eine im Wesentlichen senkrecht zu der Rohrachse erstreckte Verbindungswand 13 angeschlossen ist. Die freie umlaufende Kante der Mantelwand 12 ist durch eine Stirnwand 14 abgeschlossen. In dem dargestellten Ausführungsbeispiel ist das Kopfstück 6 im Wesentlichen kreiszylindrisch ausgebildet, sodass die Stirn-wand 14 eine Kreisfläche ausfüllt.

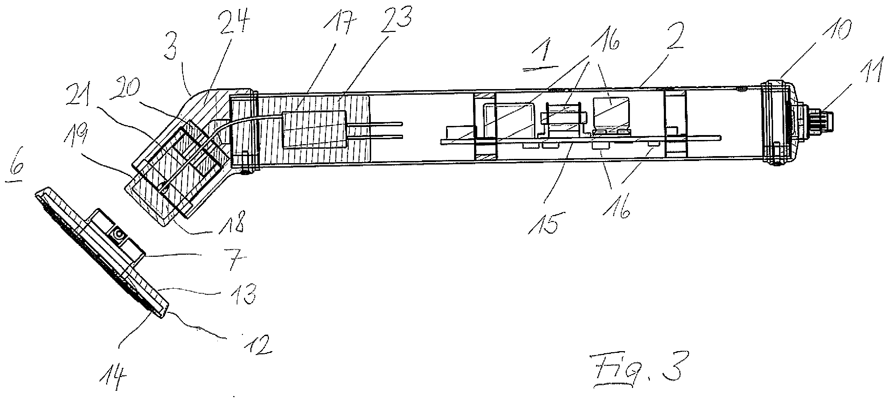

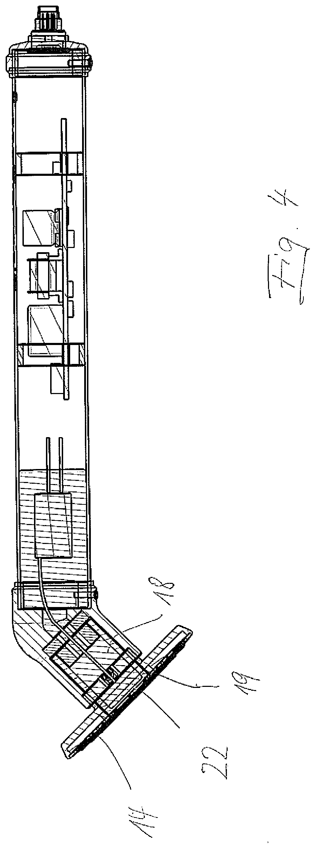

Die Figuren 3 und 4 lassen den inneren Aufbau des Gehäuses 1 erkennen, wobei in Figur 3 das Kopfstück 6 separat dargestellt ist, während in Figur 4 das Kopfstück 6 über die Rastverbindung aus Raststift 8 und Rastöffnung 9 an das Winkelstück 3 angekoppelt ist.

In den Figuren 3 und 4 ist schematisch dargestellt, dass sich in dem Innenraum des Griffstücks 2 eine Platine 15 befindet, auf der sich Bauelemente 16 für eine elektrische Steuerung befinden. Die Platine 15 ist an eine Netz-Wechselspannung über ein (nicht dargestelltes) Kabel angeschlossen, das durch die Kabeldurchführung 11 in der Stirnkappe 10 des Gehäuses 1 zugeführt wird. Über eine nicht näher dargestellte Verbindung wird eine gleichgerichtete Spannung einem Inverter 17 zugeführt, der in bekannter Weise zur Erzeugung einer Hochspannung geeignet ist, die als Wechsel-Hochspannung einer massiven, zylindrischen Elektrode 18 zugeführt wird. Die Elektrode 18 ist von einem topfförmigen Dielektrikum 19 umschlossen, das im Innern des Winkelstücks 3 an eine isolierende Durchführung 20 für eine Hochspannungsleitung 21 anschließt.

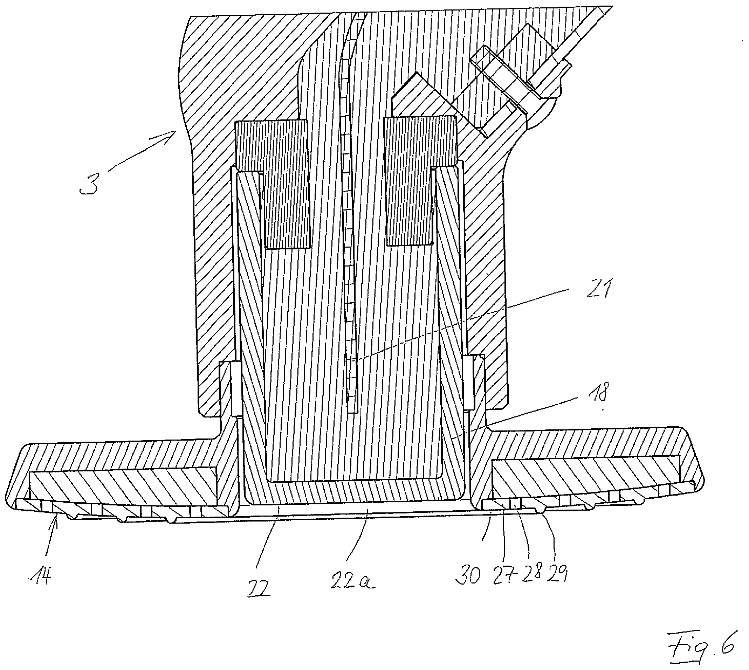

Wie Figur 4 verdeutlicht, erstreckt sich die Elektrode 18 mit dem Dielektrikum 19 in den Bereich der Stirnwand 14 soweit hinein, dass zwischen der Unterseite des Bodens 22 und der Unterseite der Stirnwand 14 ein geringer Zwischenraum verbleibt, der in Figur 6 deutlicher dargestellt ist.

Die Figuren 3 und 4 lassen erkennen, dass der Bereich der Schaltung, in dem die Hochspannung erzeugt wird und die Hochspannung zur Elektrode 18 weitergeleitet wird, im Innern des Griffstücks 2 und des Winkelstücks 3 vollständig mit einer Vergussmasse 23, 24 ausgefüllt ist, um eine zusätzliche Sicherheit gegen einen Hochspannungsüberschlag zu gewährleisten.

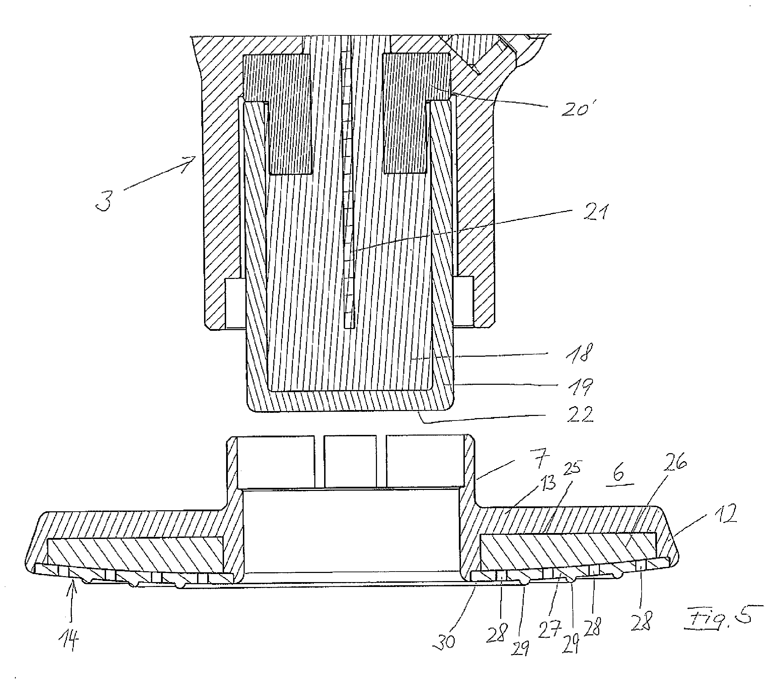

Die Figuren 5 und 6 verdeutlichen den konkreten Aufbau des Kopfstücks 6, der Mantelwand 12 und der Stirnwand 14 in Relation zum Ende des Winkelstücks 3 in separater Darstellung und im montierten Zustand. Dabei wird deutlich, dass sich der rohrförmige Ansatz 7 bis zur Stirnwand 14 erstreckt, sodass durch den rohrförmigen Ansatz 7, die Verbindungswand 13, die Mantelwand 12 und die Stirnwand 14 eine ringförmige Vorratskammer 25 begrenzt wird, die mit einem mit einem Behandlungsmittel getränkten Trägermaterial 26 in Form eines schwammförmigen Ringkörpers ausgefüllt ist. Wie insbesondere Figur 6 erkennen lässt, ist die Stirn-wand 14 zentral durch den Boden 22 des Dielektrikums 19 als erster Teil der Stirnwand 14 gebildet, während sich nach radial außen ein zweiter Teil der Stirnwand 14 anschließt, der aus einem ringförmigen flächigen Wandstück 27 besteht, das radial innen von dem rohrförmigen Ansatz 7 und radial außen von der Mantelwand 12 gehalten wird. Dieses flächige Wandstück 27 besteht in dem dargestellten Ausführungsbeispiel aus einem isolierenden Kunststoff und weist zahlreiche Durchgangsöffnungen 28 auf, die in ihrer Anordnung auf der Stirnwand 14 in Figur 1 erkennbar sind. Auf der Unterseite des Wandstücks 27 befinden sich ferner angeformte ringförmige Rippen, die als Abstandshalter 29 dienen. Das Wandstück 27 besteht aus einem flexiblen Material, das sich durch einen Andruck an eine (nicht dargestellte) zu behandelnde Oberfläche in den Bereich der Vorratskammer 25 hin-ein verformen lässt, sodass das Volumen der Vorratskammer 25 und des darin befindlichen Trägermaterials verkleinert wird. Durch das Zusammendrücken des Trägermaterials wird das darin enthaltene Behandlungsmittel durch die Durchgangsöffnungen 28 hindurchgedrückt und gelangt in den Bereich der zu behandelnden Oberfläche zwischen den Abstandshaltern 29. Durch die Abstandshalter 29 ergibt sich eine gegenüber den Abstandshalter 29 zurückgesetzte Oberfläche 30 des Wandstücks 27, die mit der Unterseite des Bodens 22 des Dielektrikums 19 fluchtet. Die Höhe der Abstandshalter 29 bestimmt daher die Höhe des Zwischenraums 22a zwischen dem Boden 22 des Dielektrikums 19 und der zu behandelnden Oberfläche. In diesem Zwischenraum bildet sich das dielektrisch behinderte Plasma aus, wenn an die Elektrode 18 eine Hochspannung angelegt wird, wobei die zu behandelnde Oberfläche, beispielsweise die Hautoberfläche eines menschlichen oder tierischen Körpers, als floatende Gegenelektrode dient. Selbstverständlich schließt die Erfindung auch nicht aus, dass der Körper - und damit die zu behandelnde Oberfläche geerdet werden, um ein Floaten des Potentials der zu behandelnden Oberfläche als Gegenelektrode zu verringern.

Es ist erkennbar, dass bei diesem Ausführungsbeispiel das Dielektrikum 19 eine starre Elektrode 18 eng umfasst, sodass die Stirnwand 14 im Bereich des Dielektrikums 19 unflexibel ist. Das Zentrum der Stirnwand ist somit starr, während der das Dielektrikum 19 umgebende zweite Teil der Stirnwand 14 in Form des Wandstücks flexibel ist und sich an Konturen der zu behandelnden Oberfläche in Grenzen anpassen kann. Die Verkleinerung des Volumens der Vorratskammer 25, durch die das Behandlungsmittel durch die Durchgangsöffnungen 28 in den Bereich der zu behandelnden Oberfläche gedrückt wird, erfolgt durch eine Ausbeulung des Wandstücks 27 in das Innere der Vorratskammer 25 hinein, wenn durch das Gehäuse 1 mit dem Griffstück 2 ein Behandlungsdruck auf die zu behandelnde Oberfläche ausgeübt wird. Das Volumen der Vorratskammer 25 ist so gewählt, dass die für eine vorgesehene Behandlung, beispielsweise eine kosmetische Gesichtsbehandlung, erforderliche Menge an Behandlungsmittel durch die Durchgangsöffnungen 28 während der Behandlung ausdrückbar ist. Nach dem Ende der Behandlung kann das Kopfstück 6 über die Raststifte 8 von dem Winkelstück 3 abgenommen und durch ein neues, mit einem geeigneten Behandlungsmittel gefülltes Kopfstück 6 ersetzt werden, sodass das Kopfstück 6 als Einmalteil verwendbar ist und anschließend entsorgt wird. Es ist erkennbar, dass lediglich die glatte Unterseite des Dielektrikums 19 gereinigt werden muss, was aufgrund der glatten Fläche jedoch unproblematisch möglich ist.

Die Figuren 5 und 6 lassen noch erkennen, dass das flexible Wandstück 27 in etwas vorgewölbter Form montiert sein kann, wodurch schon bei geringem Behandlungsdruck ein Eindrücken in das Volumen der Vorratskammer 25 erfolgt und bereits am Beginn der Behandlung das Behandlungsmittel durch die Durchgangsöffnungen 28 in den Bereich der zu behandelnden Oberfläche gedrückt wird.

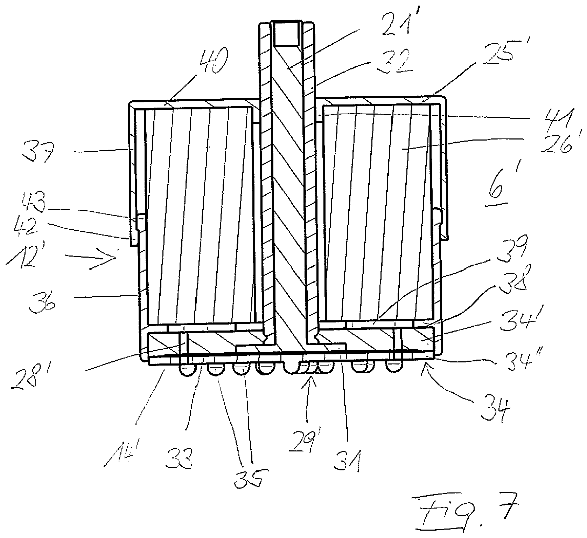

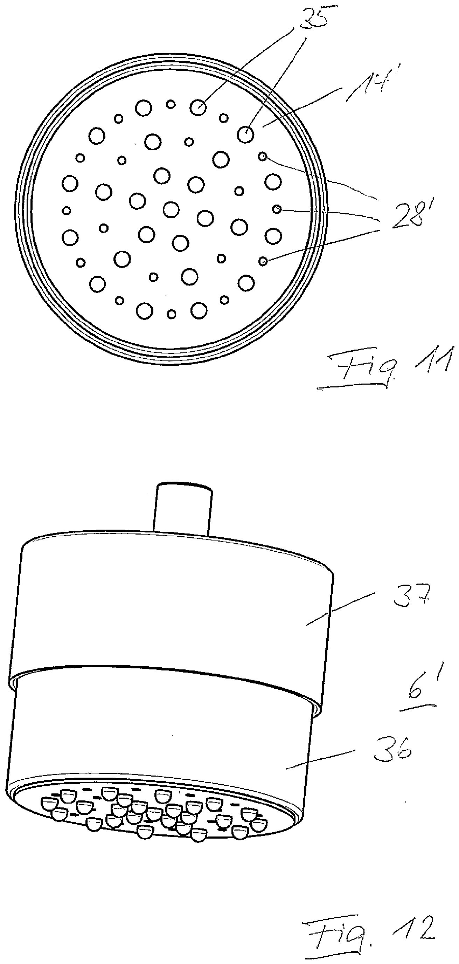

In den Figuren 7 bis 12 ist ein Kopfstück 6' einer anderen Ausführungsform eines erfindungsgemäßen Gehäuses dargestellt. Figur 7 zeigt einen vertikalen Schnitt durch das Kopfstück 6'. Die Ankopplung des Kopfstücks 6' an das Winkelstück 3 erfolgt in einer modifizierten Weise über ein massives metallisches Hochspannungs-Anschlussstück 21' in Form eines zylindrischen Bolzens, der am unteren Ende in einen kreisförmigen massiven Flansch 31 übergeht. Der Bolzen des metallischen Anschlussstücks 21' ist von einem eng anliegenden, isolierenden Rohrstück 32 umgeben, das sich bis zum Flansch 31 erstreckt. An der Unterseite des Flansches 31 liegt eine flächige, flexible Elektrode 33 an, die in ein Dielektrikum 34 vollständig eingebettet ist. Das Dielektrikum erstreckt sich allseitig über den Rand der Elektrode 33 hinaus und besteht aus zwei flächigen Schichten 34', 34", die die Elektrode 33 auf beiden Oberflächen abdecken und radial außerhalb der Elektrode 33 fest miteinander verbunden sind, beispielsweise durch Verkleben oder Verschweißen. Das Dielektrikum 34 bildet eine Stirnwand 14'. Die Oberfläche der Stirnwand 14' wird durch die untere Schicht 34" des Dielektrikums gebildet, das mit vorstehenden Noppen 35 als Abstandshalter 29' versehen ist, die einstückig mit der unteren Schicht 34" des Dielektrikums ausgebildet sind. Das Dielektrikum weist eine Vielzahl von Durchgangsöffnungen 28' auf, von denen in den Schnittdarstellungen der Figuren 7 bis 9 nur zwei sichtbar sind. Die Verteilung der Durchgangsöffnungen 28' über die Stirnwand 14' wird durch die Ansicht der Figur 11 deutlich, die auch die Lage der die Abstandshalter 29' bildenden zahlreichen Noppen 35 in diesem Ausführungsbeispiel illustriert.

Die Stirnwand 14' wird an ihrem radialen Rand durch eine Mantelwand 12' begrenzt, die durch einen unteren Mantelwandabschnitt 36 und einen oberen Mantelwandabschnitt 37 gebildet wird. Die Mantelwandabschnitte 36, 37 sind kreiszylindrisch ausgebildet und greifen teleskopisch ineinander. Vom unteren Mantelwandabschnitt 36 erstreckt sich nach radial innen ein Zwischenboden 38, der auf der Oberseite des Dielektrikums 34 aufliegt. Der Zwischenboden 38 ist mit großen Durchgangsöffnungen 39 versehen. Der obere Mantelwandabschnitt 37 geht in eine horizontale Deckelwand 40 über, die sich bis zu dem isolierenden Rohr 32 erstreckt und dort in einen nach unten gerichteten rohrförmigen Abschnitt 41 über-geht, der am isolierenden Rohr 32 gleitend anliegt.

Die Mantelwandabschnitte 36, 37 greifen teleskopisch ineinander und weisen an ihren überlappenden Enden jeweils einen Ringwulst 42, 43 auf, wobei der Ringwulst 42 an dem oberen, äußeren Mantelwandabschnitt 37 nach innen gerichtet und der Ringwulst 43 am Ende des inneren, unteren Mantelwandabschnitts 36 nach außen gerichtet ist. Demzufolge verhindern die Ringwülste 42, 43, dass die Mantelwandabschnitte 36, 37 über die in Figur 7 dargestellte Ausgangsstellung hinaus im Behandlungsbetrieb voneinander abgezogen werden.

Durch die Deckelwand 40 mit dem rohrförmigen Abschnitt 41, die teleskopisch ineinander greifenden Mantelwandabschnitte 36, 37 und den Zwischenboden 38 wird eine ringförmige Vorratskammer 25' begrenzt, in der wiederum ein Trägermaterial 26' angeordnet ist, das mit einem Behandlungsmittel getränkt ist. Durch einen Druck auf die (nicht dargestellte) zu behandelnde Oberfläche, an der die Noppen 35 als Abstandshalter 29' anliegen, wird der obere Mantelwandabschnitt 37 relativ zu dem unteren Mantelwandabschnitt 36 nach unten bewegt, sodass das Volumen der Vorratskammer 25' verkleinert und das Trägermaterial 26' zusammengedrückt wird. Dadurch gelangt Behandlungsmaterial aus dem Trägermaterial 26' durch die Durchgangsöffnungen 39 des Zwischenbodens 38 und die Durchgangsöffnungen 28' des Dielektrikums hindurch in den Bereich der zu behandelnden Oberfläche, der durch die Abstandshalter 35 zwischen der zu behandelnden Oberfläche und der gegenüber den Abstandshalter 29' zurückgesetzte Oberfläche 30' als Luftraum frei gehalten wird und in dem sich das dielektrisch behinderte Plasma ausbilden kann.

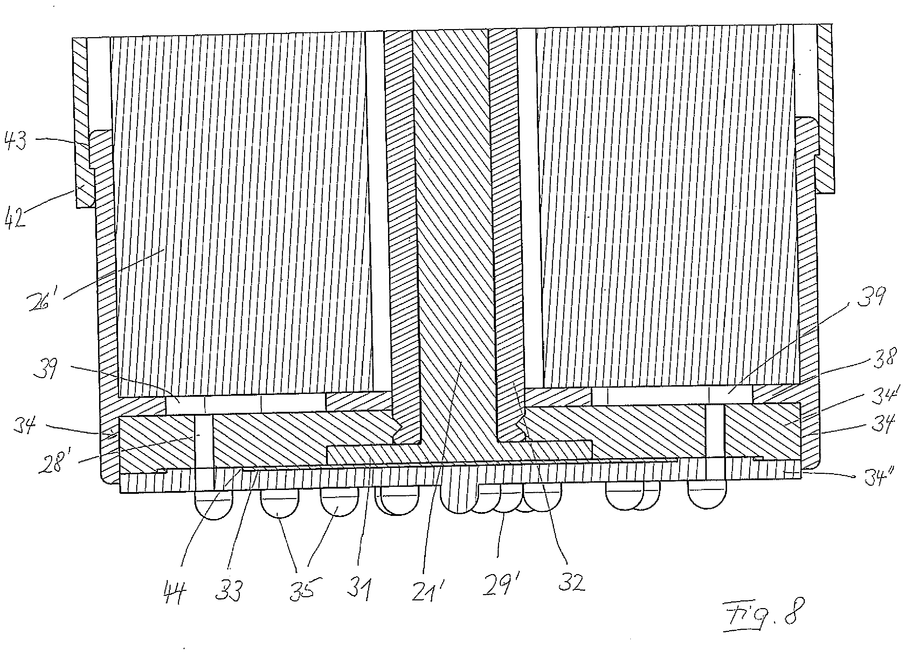

Insbesondere Figur 8 verdeutlicht, dass mit den Durchgangsöffnungen 28' des Dielektrikums 34 wesentlich größere Durchgangsöffnungen 44 der Elektrode 33 fluchten. Beim Verbindung der beiden Schichten 34', 34" des Dielektrikums füllt dieses die Durchgangsöffnungen 44 der Elektrode 33 bis zur lichten Weite der Durchgangsöffnung 28' aus, sodass sich ein Durchgangskanal ergibt, der durchgehend vom Dielektrikum 34 begrenzt ist, wodurch ein unmittelbarer Kontakt zwischen dem Behandlungsmittel und der Elektrode 33 unmöglich wird.

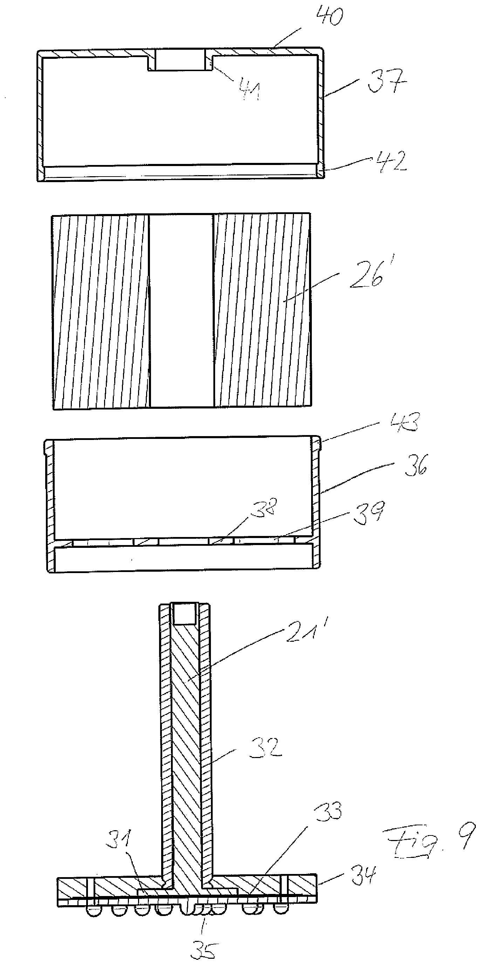

Figur 9 verdeutlicht die Einzelteile des Kopfstücks 6' in explodierter Darstellung.

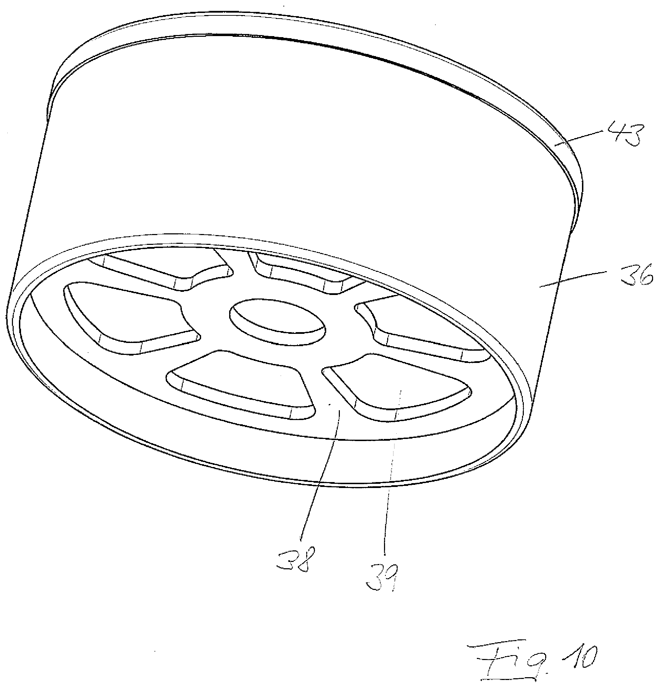

Figur 10 zeigt ein Spritzgussteil mit dem unteren Mantelwandabschnitt 36, dem Ringwulst 43, dem Zwischenboden 38 und verdeutlicht die Größe der Durchgangsöffnungen 39, die einen Austritt des Behandlungsmittels aus der Vorratskammer 25' nicht behindern sollen.

Figur 12 zeigt eine perspektivische Ansicht von schräg unten auf das Kopfstück gemäß dieser Ausführungsform, bei der der obere Mantelwandabschnitt 37 gegenüber dem unteren Mantelwandabschnitt 36 nach unten verschiebbar ist, sodass das Volumen der Vorratskammer 25' mittels eines Drucks auf die zu behandelnde Oberfläche verkleinert wird.

In einer anderen Ausführungsform könnte das Kopfstück 6' mit einer einheitlichen Mantelwand ausgebildet sein, die jedoch nach radial innen eindrückbar ist. Dadurch könnte die Verkleinerung des Volumens der Vorratskammer 25' durch einen nach radial innen gerichteten Fingerdruck auf die Mantelwand bewirkt werden. Auch auf diese Weise könnte das Behandlungsmittel aus der Vorratskammer 25' in dem Bereich der zu behandelnden Oberfläche ausgedrückt werden. Eine Variante dieser Ausführungsform kann alternativ oder ergänzend auch eine flexible Deckelwand 40 vorsehen.

Auch das Kopfstück 6' kann als Auswechselteil nach einer Behandlung vorgesehen werden. Durch das prinzipiell größere Volumen der Vorratskammer 25' kann dieses Kopfstück 6' für eine deutliche größere Behandlung vorgesehen werden. Mit dem Austausch des Kopfstücks 6' kann selbstverständlich auch ein Kopfstück 6' mit einem anderen Behandlungsmittel vorgesehen werden. So ist es bei kosmetischen oder medizinischen Behandlungen möglich, zunächst mit einem sehr wirksamen Behandlungsmittel zu arbeiten und bei der Verbesserung des Ausgangszustands zu einem milderen Behandlungsmittel überzugehen. Dieser Schritt kann selbstverständlich zum Übergang eines noch milderen Behandlungsmittels wiederholt werden. Schließlich ist es möglich, als Behandlungsmittel ein nur noch pflegendes Mittel einzusetzen.

Es ist für den Fachmann ohne weiteres erkennbar, dass die dargestellten Ausführungsbeispiele unterschiedliche Mechanismen für die Verkleinerung des Volumens der Vorratskammer 25, 25'jeweils in Kombination mit einer bestimmten Ausbildung der Stirnwand 14, 14' darstellen, dass jedoch die unterschiedlichen Methoden der Volumenverkleinerung beliebig mit der einteiligen oder mehrteiligen Ausbildung der Stirnwand 14, 14' kombiniert werden können. So ist es möglich, die Ausbildung des Kopfstücks 6' mit teleskopisch ineinander verschiebbaren Mantelwandabschnitten 36, 37 bei einer Stirnwand 14 vorzusehen, bei der sich die Elektrode nur im Bereich eines zentralen Dielektrikums befindet und radial um das Dielektrikum herum ein ringförmiges Wandstück vorgesehen ist, das die Durchgangsöffnungen 28 aufweist.

In gleicher Weise kann die Stirnwand 14' komplett durch das Dielektrikum 34 gebildet sein, das jedoch durch den Druck auf die zu behandelnde Oberfläche mit der eingebetteten flexiblen Elektrode 33 verformbar ist und auf diese Weise eine Verkleinerung des Volumens der Vorratskammer 25, 25' bewirkt.

## Patentansprüche

1. Behandlungsgerät für eine mit einem dielektrisch behinderten Plasma zu behandelnde Oberfläche, mit einem Gehäuse (1), das eine Stirnwand (14, 14') aufweist, mit einer zur zu behandelnden Oberfläche durch ein wenigstens einen Teil der Stirnwand (14, 14') bildendes Dielektrikum (19, 34) abgeschirmten Elektrode (18, 33), die mit einem Hochspannungsgenerator (17) verbindbar ist, wobei die Stirnwand (14, 14') wenigstens einen Abstandshalter (29, 29') aufweist, mit dem beim Anliegen des wenigstens einen Abstandshalters (29, 29') an der zu behandelnden Oberfläche wenigstens ein Gasraum gebildet wird, in dem sich für die Behandlung das dielektrisch behinderte Plasma ausbildet, **dadurch gekennzeichnet, dass** auf der von der zu behandelnden Oberfläche abgewandten Seite der Stirnwand (14, 14') eine mit einem Behandlungsmittel füllbare ringförmige Vorratskammer (25, 25') angeordnet ist, dass die Stirnwand (14, 14') eine Vielzahl von Durchgangsöffnungen (28, 28') aufweist und dass die Vorratskammer (25, 25') in ihrem Volumen so verkleinerbar ist, dass bei der Verkleinerung des Volumens Behandlungsmittel durch die Durchgangsöffnungen (28, 28') in den Bereich der zu behandelnden Oberfläche gelangt.

2. Behandlungsgerät nach Anspruch 1, **dadurch gekennzeichnet, dass** das Volumen der Vorratskammer (25, 25') durch Ausübung eines Drucks auf die zu behandelnde Oberfläche mittels des Gehäuses (1) verkleinerbar ist.

3. Behandlungsgerät nach Anspruch 1 oder 2, **dadurch gekennzeichnet, dass** das Gehäuse (1) teleskopisch ineinander greifende Mantelwandabschnitte (36, 37) aufweist, die durch Druck auf das Gehäuse (1) in Richtung der zu behandelnden Oberfläche ineinander schiebbar sind.

4. Behandlungsgerät nach einem der Ansprüche 1 bis 3, **dadurch gekennzeichnet, dass** die Stirnwand (14, 14') wenigstens teilweise flexibel ausgebildet ist und sich durch einen Druck auf das Gehäuse (1) in Richtung der zu behandelnden Oberfläche in Richtung des Inneren der Vorratskammer (25') verformt.

5. Behandlungsgerät nach einem der Ansprüche 1 bis 4, **dadurch gekennzeichnet, dass** das Gehäuse (1) eine die Vorratskammer (25, 25') begrenzende flexible Mantelwand aufweist, die zur Verkleinerung des Volumens der Vorratskammer (25, 25') eindrückbar ist.

6. Behandlungsgerät nach einem der Ansprüche 1 bis 5, **dadurch gekennzeichnet, dass** die Elektrode (33) als flächige Elektrode in das flächig ausgebildete Dielektrikum (34) allseitig eingebettet ist und dass die Durchgangsöffnungen (28') des Dielektrikums kleiner als die entsprechenden Durchgangsöffnungen (39) der Elektrode (33) sind, sodass Durchgangskanäle gebildet sind, die durchgehend radial von dem Dielektrikum (34) begrenzt sind.

7. Behandlungsgerät nach Anspruch 6, **dadurch gekennzeichnet, dass** die Anordnung aus Dielektrikum (34) und eingebetteter Elektrode (33) flexibel ausgebildet ist.

8. Behandlungsgerät nach einem der Ansprüche 1 bis 7, **dadurch gekennzeichnet, dass** sich das Dielektrikum (34) einstückig über die gesamte Stirnwand (14') erstreckt.

9. Behandlungsgerät nach einem der Ansprüche 1 bis 7, **dadurch gekennzeichnet, dass** die Stirnwand (14) wenigstens zweiteilig ausgebildet ist.

10. Behandlungsgerät nach Anspruch 9, **dadurch gekennzeichnet, dass** ein erstes Teil der Stirnwand (14) durch das die Elektrode (18) abschirmende Dielektrikum (19) gebildet ist und wenigstens ein zweiter Teil (27) der Stirnwand die Vorratskammer (25) begrenzt und die Durchgangsöffnungen (28) aufweist.

11. Behandlungsgerät nach Anspruch 10, **dadurch gekennzeichnet, dass** das Dielektrikum (19) einen zentralen Abschnitt der Stirnwand (14) bildet und der zweite Teil (27) der Stirnwand (14) das Dielektrikum (19) ringförmig umgibt.

12. Behandlungsgerät nach Anspruch 10 oder 11, **dadurch gekennzeichnet, dass** das wenigstens eine zweite Teil (27) der Stirnwand (14) aus einem isolierenden Kunststoff besteht.

13. Behandlungsgerät nach einem der Ansprüche 10 bis 12, **dadurch gekennzeichnet, dass** der wenigstens eine zweite Teil (27) der Stirnwand (14) den wenigstens einen Abstandshalter (29) ausbildet.

14. Behandlungsgerät nach einem der Ansprüche 1 bis 13, **dadurch gekennzeichnet, dass** die zu behandelnde Oberfläche zu der durch das Dielektrikum (19, 34) abgeschirmten Elektrode (18, 33) als Gegenelektrode fungiert.

15. Behandlungsgerät nach einem der Ansprüche 1 bis 14, **dadurch gekennzeichnet, dass** ein Gehäuseteil aus Stirnwand (14, 14') und zumindest einem Teil der Vorratskammer (25, 25') als auswechselbares Kopfstück (6, 6') ausgebildet ist.

16. Behandlungsgerät nach einem der Ansprüche 1 bis 15, **dadurch gekennzeichnet, dass** das Behandlungsmittel in einem Trägermaterial (26, 26') in die Vorratskammer (25, 25') eingebracht ist.

## Claims

1. A treatment device for a surface to be treated using a dielectric barrier plasma, comprising a housing (1) which has an end wall (14, 14') and comprising an electrode (18, 33) which is shielded from the surface to be treated by a dielectric (19, 34) that forms at least one part of the end wall (14, 14') and which electrode (18, 33) can be connected to a high-voltage generator (17), wherein the end wall (14, 14') has at least one spacer (29, 29') by means of which at least one gas chamber is formed when the at least one spacer (29, 29') rests against the surface to be treated, and the dielectric barrier plasma is formed in the gas chamber for the treatment process, **characterized in that** a ring-shaped storage chamber (25, 25') which can be filled with a treatment agent is arranged on the side of the end wall (14, 14') facing away from the surface to be treated, the end wall (14, 14') has a plurality of passage openings (28, 28'), and the volume of the storage chamber (25, 25') can be reduced in such a way that treatment agent reaches the region of the surface to be treated through the passage openings (28, 28') when the volume is reduced.

2. The treatment device as claimed in claim 1, **characterized in that** the reduction of the size of the storage chamber (25, 25') takes place by applying a pressure onto the surface to be treated, by means of the housing (1).

3. The treatment device as claimed in claim 1 or 2, **characterized in that** the housing (1) comprises peripheral wall sections (36, 37) which telescopically engage into each other and which can be slid into each other by way of pressure applied onto the housing (1) in the direction of the surface to be treated.

4. The treatment device as claimed in one of claims 1 to 3, **characterized in that** the end wall (14, 14') is designed to be at least partially flexible and deforms in the direction of the interior of the storage chamber (25') by way of pressure applied onto the housing (1) in the direction of the surface to be treated.

5. The treatment device as claimed in one of claims 1 to 4, **characterized in that** the housing (1) comprises a flexible peripheral wall which delimits the storage chamber (25, 25') and can be pressed inward in order to reduce the volume of the storage chamber (25, 25').

6. The treatment device as claimed in one of claims 1 to 5, **characterized in that** the electrode (33), as a planar electrode, is embedded on all sides into the planar dielectric (34) and the passage openings (28') of the dielectric are smaller than the corresponding passage openings (39) of the electrode (33), and therefore passage channels are formed which are continuously radially delimited by the dielectric (34).

7. The treatment device as claimed in claim 6, **characterized in that** the arrangement comprising dielectric (34) and embedded electrode (33) is designed to be flexible.

8. The treatment device as claimed in one of claims 1 to 7, **characterized in that** the dielectric (34) extends as one piece over the entire end wall (14').

9. The treatment device as claimed in one of claims 1 to 7, **characterized in that** the end wall (14) is in the form of at least two parts.

10. The treatment device as claimed in claim 9, **characterized in that** a first part of the end wall (14) is formed by the dielectric (19) shielding of the electrode (18) and at least one second part (27) of the end wall delimits the storage chamber (25) and comprises the passage openings (28).

11. The treatment device as claimed in claim 10, **characterized in that** the dielectric (19) forms a central section of the end wall (14) and the second part (27) of the end wall (14) annularly surrounds the dielectric (19).

12. The treatment device as claimed in claim 10 or 11, **characterized in that** the at least one second part (27) of the end wall (14) consists of an insulating plastic.

13. The treatment device as claimed in one of claims 10 to 12, **characterized in that** the at least one second part (27) of the end wall (14) forms the at least one spacer (29).

14. The treatment device as claimed in one of claims 1 to 13, **characterized in that** the surface to be treated functions as a counterelectrode to the electrode (18, 33) shielded by the dielectric (19, 34).

15. The treatment device as claimed in one of claims 1 to 14, **characterized in that** one housing part comprising end wall (14, 14') and at least one part of the storage chamber (25, 25') is designed as an exchangeable headpiece (6, 6').

16. The treatment device as claimed in one of claims 1 to 15, **characterized in that** the treatment agent is introduced into the storage chamber (25, 25') in a carrier material (26, 26')

## Revendications

1. Appareil de traitement pour une surface à traiter avec un plasma empêché par voie diélectrique, comportant un boîtier (1) qui comprend une paroi frontale (14, 14'), une électrode (18, 33) qui est protégée vis-à-vis de la surface à traiter par un diélectrique (19, 34) formant au moins une partie de la paroi frontale (14, 14') et qui est susceptible d'être connectée à un générateur haute tension (17),
dans lequel
la paroi frontale (14, 14') comprend au moins un élément écarteur (29, 29') permettant de former au moins une chambre à gaz lors de l'appui dudit au moins un élément écarteur (29, 29') contre la surface à traiter, chambre dans laquelle se forme le plasma empêché par voie diélectrique, en vue du traitement,
**caractérisé en ce que**
sur le côté de la paroi frontale (14, 14') détourné de la surface à traiter, il est prévu une chambre de réserve (25, 25') annulaire remplissable avec un produit de traitement, **en ce que**
la paroi frontale (14, 14') présente une multitude d'ouvertures traversantes (28, 28'), et **en ce que**
la chambre de réserve (25, 25') est susceptible d'être réduite en volume de telle sorte que lors de la réduction du volume le produit de traitement parvient à travers les ouvertures traversantes (28, 28') jusque dans la zone de la surface à traiter.

2. Appareil de traitement selon la revendication 1,
**caractérisé en ce que**
le volume de la chambre de réserve (25, 25') est susceptible d'être réduit par l'application d'une pression sur la surface à traiter au moyen du boîtier (1).

3. Appareil de traitement selon la revendication 1 ou 2,
**caractérisé en ce que**
le boîtier (1) présente des portions de paroi enveloppe (36, 37) qui s'engagent mutuellement de façon télescopique et qui peuvent être emboîtées l'une dans l'autre par l'application d'une pression sur le boîtier (1) en direction de la surface à traiter.

4. Appareil de traitement selon l'une des revendications 1 à 3,
**caractérisé en ce que**
la paroi frontale (14, 14') est réalisée au moins partiellement de façon flexible et se déforme en direction de l'intérieur de la chambre de réserve (25') par une pression appliquée sur le boîtier (1) en direction de la surface à traiter.

5. Appareil de traitement selon l'une des revendications 1 à 4,
**caractérisé en ce que**
le boîtier (1) présente une paroi enveloppe flexible qui délimite la chambre de réserve (25, 25') et qui susceptible d'être enfoncée pour réduire le volume de la chambre de réserve (25, 25').

6. Appareil de traitement selon l'une des revendications 1 à 5,
**caractérisé en ce que**
l'électrode (33) est noyée sous forme d'électrode surfacique de tous les côtés dans le diélectrique (34) réalisé de manière surfacique, et **en ce que** les ouvertures traversantes (28') du diélectrique sont plus petites que les ouvertures traversantes correspondantes (39) de l'électrode (33), de manière à former des canaux traversants qui sont délimités radialement en continu par le diélectrique (34).

7. Appareil de traitement selon la revendication 6,
**caractérisé en ce que**
l'agencement du diélectrique (34) et de l'électrode noyée (33) est réalisé de façon flexible.

8. Appareil de traitement selon l'une des revendications 1 à 7,
**caractérisé en ce que**
le diélectrique (34) s'étend d'un seul tenant sur toute la paroi frontale (14').

9. Appareil de traitement selon l'une des revendications 1 à 7,
**caractérisé en ce que**
la paroi frontale (14) est réalisée au moins en deux parties.

10. Appareil de traitement selon la revendication 9,
**caractérisé en ce que**
une première partie de la paroi frontale (14) est formée par le diélectrique (19) protégeant l'électrode (18) et au moins une seconde partie (27) de la paroi frontale délimite la chambre de réserve (25) et présente les ouvertures traversantes (28).

11. Appareil de traitement selon la revendication 10,
**caractérisé en ce que**
le diélectrique (19) constitue une portion centrale de la paroi frontale (14) et la seconde partie (27) de la paroi frontale (14) entoure en forme annulaire le diélectrique (19).

12. Appareil de traitement selon la revendication 10 ou 11,
**caractérisé en ce que**
ladite au moins une seconde partie (27) de la paroi frontale (14) est constituée en matière plastique isolante.

13. Appareil de traitement selon l'une des revendications 10 à 12,
**caractérisé en ce que**
ladite au moins une seconde partie (27) de la paroi frontale (14) forme ledit au moins un élément écarteur (29).

14. Appareil de traitement selon l'une des revendications 1 à 13,
**caractérisé en ce que**
la surface à traiter fait office d'électrode complémentaire à l'électrode (18, 33) protégée par le diélectrique (19, 34).

15. Appareil de traitement selon l'une des revendications 1 à 14,
**caractérisé en ce que**
une partie du boîtier constituée par la paroi frontale (14, 14') et par une partie au moins de la chambre de réserve (25, 25') est réalisée sous forme de partie de tête interchangeable (6, 6').

16. Appareil de traitement selon l'une des revendications 1 à 15,
**caractérisé en ce que**
le produit de traitement est introduit dans la chambre de réserve (25, 25') en étant dans un matériau porteur (26, 26').
